Europäisches Patentamt

European Patent Office

Office européen des brevets

(11) Publication number: **0 354 196**
**A1**

# EUROPEAN PATENT APPLICATION

(21) Application number: **89850202.6**

(22) Date of filing: **19.06.89**

(51) Int. Cl.5: **A 61 F 13/15**

(30) Priority: **29.06.88 SE 8802448**

(43) Date of publication of application:
**07.02.90 Bulletin 90/06**

(84) Designated Contracting States: **GR**

(71) Applicant: **Mölnlycke AB**
**S-405 03 Göteborg (SE)**

(72) Inventor: **Nyberg, Anita**
**Lövskogsvägen 57**
**S-435 37 Mölnlycke (SE)**

(74) Representative: **Hammar, Ernst et al**
**H. ALBIHNS PATENTBYRA AB P.O. Box 3137**
**S-103 62 Stockholm (SE)**

(54) **Absorbent products.**

(57) The present invention relates to an absorbent product, such as a diaper, a sanitary napkin or an incontinence guard, including a cellulose fluff-pulp absorption pad which is comprised of two mutually separate material layers (3, 4), of which the inner pad layer (3), intended to lie nearest the wearer's body in use, is soft, has slight flexural rigidiy and a bulk density of more than 13 cm$^3$/g, and the outer pad layer (4), in relation to the wearer, is narrower than the inner pad layer and is adapted to the width of the wearer's crotch, and is heavily compacted and has a bulk density of less than 9 cm$^3$/g.

According to the invention, the product is characterized in that a sheet (5) of non-woven material is attached to the whole or parts of the outer surface of the inner pad layer (3).

FIG. 2

EP 0 354 196 A1

# Description

## Absorbent products

The present invention relates to an absorbent product, such as a diaper, sanitary napkin or an incontinence guard, which includes an absorbent pad which is made of cellulose fluff pulp and which is comprised of two mutually separate layers, of which the inner layer, which in use is intended to lie nearest the wearer's body, is soft and has slight flexural rigidity and a bulk density of more than 13 cm$^3$/g, and of which the other layer, the outer layer, is narrower than the inner layer and is adapted to the width of the wearer's crotch, and is heavily compacted and has a bulk density of less than 9 cm$^3$/g.

A product of this kind is known from our Swedish patent application No. 8400418-2 and the outer, compacted layer imparts a high degree of shape stability to the product, this stability being sustained even when the product is wet. Furthermore, practically all liquid delivered by the wearer to the product is taken up in the outer pad layer, due to the capillary action engendered in use. The purpose of the inner pad layer is to allow liquid to pass quickly through the outer pad layer and, from the aspect of absorption, the inner pad is operative to prevent lateral leakage of liquid from the outer pad layer and subsequent rewetting of the wearer.

Because the inner pad layer of the aforesaid product is not intended to take-up the liquid delivered by the wearer, but instead has the main function of forming the surface contact with the wearer's body and of preventing contact of the outer layer with the wearer, the inner layer can be made very thin, as seen from a functional aspect. However, it has been found that an inner pad layer whose thickness will satisfy all of the aforesaid functional requirements lacks sufficient inherent shape stability and that cracks or crevices tend to form in the inner pad layer during the use of the product. Cracks or crevices are also liable to form in the outer layer at times, therewith greatly impairing the spread and dispersal of liquid to the outer pad layer and making it impossible to utilize the full absorption capacity of the product.

The object of the present invention is to improve the shape or form stability of the inner pad layer of a product of the aforementioned kind, and to reduce the risk of cracks forming in the outer pad layer.

These objects are achieved in accordance with the invention, by attaching a reinforcing sheet of non-woven material to the whole or parts of the outer surface of the inner pad layer. One of the advantages afforded by the provision of such a reinforcing sheet is that the inner pad layer can be made thinner than is normally the case, such that liquid delivered by the user will reach the outer pad layer more quickly than in the case when no such sheet is provided, therewith enabling the available absorptive capacity of the product to be utilized more rapidly and, in turn, reducing the risk of leakage. Furthermore, the provision of such a reinforcement sheet will reduce the risk of small quantities of liquid from reaching the outer pad layer, for instance such liquid emissions as droplets of menstrual fluid, or droplets or urine in the case of more lenient incontinence, since such small quantities as these will remain in the inner pad layer, due to the fact that no capillary action will be engendered in such cases. Another advantage is that by attaching the side margins of the non-woven sheet to the pad enveloping sheets, or pad envelope, the outer pad layer becomes more or less enclosed, which will reduce the risk of lateral displacement of the outer pad layer and therewith also of rewetting, and in those instances when the outer pad layer includes superabsorbent particles, will also reduce the risk of particles located on or near the inner surface of the outer pad layer from migrating to the inner pad layer.

An exemplifying embodiment of the invention will now be described with reference to the accompanying drawing, in which:

Figure 1 is a view of an inventive diaper from above, and

Figure 2 is a cross-sectional view taken on the line II-II in Figure 1.

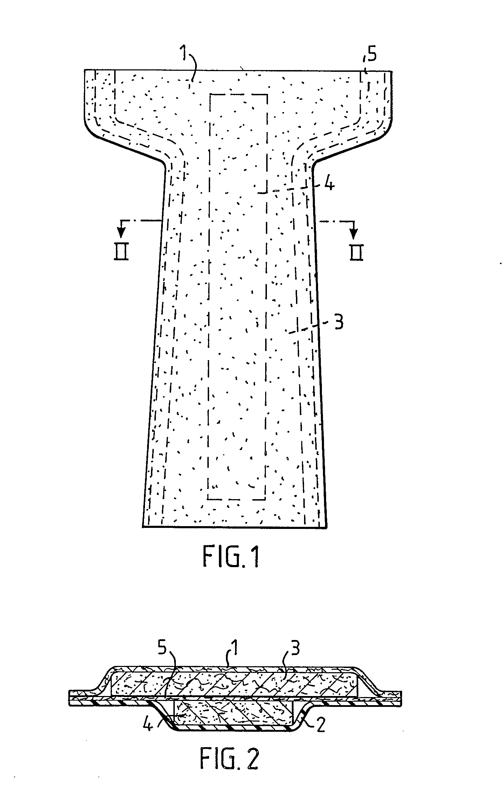

The diaper illustrated in Figures 1 and 2 includes a cellulose fluff-pulp absorption pad enclosed in an envelope comprising an inner, liquid permeable layer 1 which is intended to lie against the wearer's body in use, and an outer backing sheet or layer 2 made of liquid impermeable material. The fluff pulp is preferably a mechanical pulp, which may optionally be thermally treated, so-called TMP, or both a chemical pulp and a thermally treated pulp, so-called CTMP. The processes by which mechanical pulp is produced are more friendly to the environment than those processes employed in the manufacture of chemical pulp, and they also better utilize the wood starting material. The absorption pad is comprised of an inner T-shaped material layer 3 having a bulk density greater than 13 cm$^3$/g, and a narrower, outer material layer 4, which is heavily compacted and has a bulk density of less than 9 cm$^3$/g. The inventive absorption pad also includes a sheet 5 of non-woven material, which is attached between the two pad layers 3 and 4 and extends laterally beyond the pad layer 3 and in between the two enveloping sheets 1, 2, to which the outwardly projecting parts of the sheet 5 are attached.

The sheet 5 preferably comprises a printed bond material of rayon fiber, although a thin spunbond material may also be used. In addition to being attached to the enveloping sheets, the sheet 5 is also attached to the inner pad layer 3. Because the sheet 5 will ensure the shape stability of the inner pad layer, said layer need not be made thicker than that required to provide suitable cushioning against the wearer's body, therewith avoiding in particular chaffing around the thighs and waist from the elastications (not shown) normally incorporated in diapers of this kind. Furthermore, the sheet 5 is preferably made of an absorbent material, such as rayon, in order to assist in the prevention of rewetting of the liquid absorbed in the outer pad layer when said layer is subjected to external pressure. A non-absorbent sheet will also reduce the risk of rewetting, however, since such a sheet will improve the distribution of external forces through the product. The sheet 5 may advantageously also be attached to the outer pad layer, to this end.

Since liquid delivered by the wearer is distributed and absorbed in the outer pad layer and is delivered to the upper part of the diaper in use, it is not necessary for the sheet 5 to extend along the whole longitudinal extension of the diaper, and it will suffice to attach the sheet 5 solely to the upper half of the diaper.

When the outer pad layer 4 includes uniformly distributed particles of superabsorbent material, the sheet 5 will prevent those particles located on or in the vicinity of the inner surface of the outer pad layer from migrating to the inner pad layer 3.

In order for a product of the aforesaid kind to be able to function in the manner intended, it is necessary for the liquid delivered by the wearer to reach the outer pad layer, and preferably as quickly as possible, in order that the liquid can be distributed and spread throughout the outer pad layer in a manner to utilize the absorption capacity of the layer to the full, and so that there is engendered a capillary action which will draw liquid by suction in from the inner pad layer which has large capillaries, to the outer pad layer, which has smaller capillaries, such that the inner pad layer will become quickly dry. As before indicated, no capillary action is engendered when the quantities of liquid delivered are small and thereby absorbed in the inner pad layer without reaching the outer pad layer. In such cases, the inner pad layer will remain moist and the diaper will be uncomfortable to the wearer. Naturally, the thinner the inner pad layer the less likely this phenomenom will occur, and the risk of such an occurrence is still further reduced when the sheet 5 comprises an absorbent material and, consequently, does not impede the transport of liquid from the inner pad layer 3 to the outer pad layer 4.

Although the exemplifying embodiment has been described with reference to a diaper, it will be understood that the invention can be applied to other kinds of absorbent products, such as sanitary napkins and incontinence guards.

## Claims

1. An absorbent product, such as a diaper, a sanitary napkin or an incontinence guard, comprising a cellulose fluff-pulp absorbent body which comprises two mutually separate material layers, of which the inner layer (3) is intended to lie nearest the wearer's body in use and is soft, has slight flexural rigidity and has a bulk density of more than 13 cm$^3$/g, and the other layer (4), the outer layer relative to the wearer, is narrower than the inner pad layer and is adapted to the width of the wearer's crotch, and is highly compacted and has a bulk density of less than 9 cm$^3$/g, characterized in that a sheet (5) of non-woven material is artached to the whole of or parts of the outer surface of the inner pad layer (3).

2. A product according to claim 1, characterized in that the sheet (5) of non-woven material comprises an absorbent printed bond material, preferably rayon.

3. A product according to claim 1, characterized in that the non-woven sheet comprises a spunbonded material.

4. A product according to claim 1 and 2 or 3, characterized in that the sheet (5) is also attached to the outer pad layer (4).

5. A product according to any one of the preceding claims, characterized in that the outer pad layer (4) includes particles of a superabsorbent material.

6. A product according to any one of the preceding claims, in which the absorbent body is enveloped in a casing comprising two enveloping layers (1, 2), which extend beyond the contours of the absorption pad, characterized in that the sheet (5) of ·non-woven material, at least in the width extension of the product, extends beyond the contours of the absorption pad and is there attached to the two enveloping layers.

FIG.1

FIG. 2

European Patent
Office

EUROPEAN SEARCH REPORT

Application number

EP 89 85 0202.6

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| A | EP-A2-0 217 766 (MÖLNLYCKE AB)<br><br>--- | 1-6 | A 61 F 13/15 |
| A | DE-A-1 815 757 (KIMBERLY-CLARK CORP.)<br>*see page 2, first paragraph*<br><br>--- | 1-6 | |
| A | US-A-4 540 414 (MARVIN WISHMAN)<br><br>--- | 1-6 | |
| A | GB-A-2 111 836 (KIMBERLY-CLARK CORPORATION)<br><br>--- | 1-6 | |
| A | EP-A2-0 202 125 (THE PROCER & GAMBLE COMPANY)<br><br>--- | 1-6 | TECHNICAL FIELDS SEARCHED (Int. Cl.4) |
| A | EP-A2-0 158 914 (KIMBERLY-CLARK CORPORATION)<br><br>--- | 1-6 | A 61 F<br>A 41 B |
| A | WO-A1-83/02054 (KAMME CARL GUSTAF)<br><br>----- | 1-6 | |

The present search report has been drawn up for all claims

| Place of search | Date of completion of the search | Examiner |
|---|---|---|
| STOCKHOLM | 18-08-1989 | JÖNSSON H.C. |